# EUROPÄISCHE PATENTSCHRIFT

(11) **EP 1 523 305 B1**
(45) Veröffentlichungstag und Bekanntmachung des Hinweises auf die Patenterteilung: **31.08.2011**
(21) Anmeldenummer: 02792915.7
(22) Anmeldetag: 06.12.2002
(51) Int. Cl.: A61K 9/70

(54) **VORRICHTUNG UND PFLASTER ZUR KONTROLLIERTEN FREISETZUNG VON VANILLIN**
DEVICE AND PATCH FOR CONTROLLING THE RELEASE OF VANILLIN
DISPOSITIF ET TIMBRE POUR LA LIBERATION CONTROLEE DE VANILLINE

(30) Priorität: 07.12.2001 DE 10160110
(43) Veröffentlichungstag der Anmeldung: 20.04.2005
(73) Patentinhaber: Heilein, Ernst-Peter, 85716 Unterschleissheim (DE)
(72) Erfinder: Hoenzelaer, Norbert, 47533 Kleve (DE)
(74) Vertreter: Heilein, Ernst - Peter
(86) Internationale Anmeldenummer: PCT/EP2002/013820
(87) Internationale Veröffentlichungsnummer: WO 2003/047557

(56) Entgegenhaltungen:
- WO-A-97/03658
- WO-A-98/17262
- DE-A- 3 216 609
- DE-A- 4 433 191
- DE-A- 19 712 359
- US-A- 5 455 043
- DATABASE WPI Section Ch, Week 198342 Derwent Publications Ltd., London, GB; Class A96, AN 1983-790892 XP002239295 & JP 58 054956 A (NITTO ELECTRIC IND CO), 1. April 1983 (1983-04-01)
- DATABASE WPI Section Ch, Week 200158 Derwent Publications Ltd., London, GB; Class B04, AN 2001-528055 XP002239519 & KR 2001 025 288 A (CHOI J S), 6. April 2001 (2001-04-06)

## Beschreibung

Die vorliegende Erfindung betrifft eine Vorrichtung in Form eines Pflasters zur kontrollierten Freisetzung von Vanillin, Ethylvanillinderivaten oder deren Mischungen, sowie die Verwendung derselbigen.

Viele Aromen entfalten ausgeprägte Wirkungen an verschiedenen Rezeptoren von Neurotransmittern (GABA, NMDA, KA, nACh). Duftstoffe wie Geraniol ("Rose") und Essigsäurebutylester ("Frucht") stimulieren den GABA-Rezeptor. Hochdosiert wirken solche Rezeptorstimulantien sogar hypnotisch und anästhesierend. Vanillin hingegen hemmte ähnlich wie Coffein diesen Rezeptor. Da nach heutiger Erkenntnis die wichtigsten physiologischen Wirkungen des Coffeins auf dieser Hemmung beruhen, könnte Vanillin ähnlich stimulieren wie Coffein. Vanillin hemmte außerdem den nicotinischen Acetylcholin-Rezeptor (nACh), eine Eigenschaft, die es mit dem Schlangengift Kobratoxin oder dem Pfeilgift Curare teilt. Auch gegenüber den Glutamat-Rezeptoren (NMDA, KA) wirkte Vanillin hemmend. Dieser Effekt ist prinzipiell vergleichbar mit dem des Phencyclidins, das auch als Rauschmittel *("Angel Dust")* bekannt ist.

Neben dieser Rezeptorhemmung beeinflusst Vanillin im Zentralnervensystem auch das Verlangen, Nahrung aufzunehmen. In diesem Zusammenhang wird vermutet, dass das Vanillin die Konzentration des Neurotransmitters Serotonin im Gehirn zu Erhöhen vermag. Eine erhöhte Serotoninkonzentration im Gehirn jedoch führt nachweislich zu einem verminderten Verlangen, Nahrung aufzunehmen.

Da Serotonin nicht synthetisch hergestellt werden kann und eine Aufnahme dieses Neurotransmitters in das Gehirn aufgrund der dort lokalisierten Schrankensysteme ohnehin kaum möglich ist, wurde nun versucht, eine Erhöhung der Serotoninkonzentration im Gehirn durch die Verwendung des Duftstoffes Vanillin zu erzielen.

Eine Aufnahme dieses Duftstoffes ist aufgrund seiner lipophilen Eigenschaften über die Blut-Hirn-Schranke möglich. Als zweiter Aufnahmepfad neben der Blut-Hirn-Schranke bietet sich der Riechnerv (*Bulbus olfactorius*) an, der einen transneuralen Transport ins Gehirn erlaubt (Neurotoxicology and Teratology 1990/12/S.455-459). Vanillin kann demnach über die Nase in das Zentralnervensystem gelangen. In der Patentliteratur wurden demnach zahlreich Vorrichtungen beschrieben, mit deren Hilfe dieser Duftstoff im Körpernahen Bereich verdampft und so kontinuierlich eingeatmet werden kann (Aufnahme des Vanillins über den Riechnerv) oder mit denen Vanillin kontinuierlich dem Blutkreislauf zugeführt werden kann (Aufnahme über die Blut-Hirn-Schranke).

So beschreibt die EP-A-645 081 ein**e** Vorrichtung zu**r** Bekämpfung von Insekten umfassend einen Behälter, welcher mit einer für gasförmige etherische Öle undurchlässigen Folie bedeckt ist. Die in diesem Behälter enthaltenen insektiziden Zusammensetzungen treten nach dem Entfernen einer Schutzschicht kontinuierlich durch die Folie hindurch aus dem Behälter aus. Der Nachteil dieser Behälter liegt darin, dass sie nicht zur kontinuierlichen Freisetzung von Vanillin im Körpernahen Bereich geeignet sind.

Zur Vermeidung dessen beschreibt die EP-A-844 872 (korrespondierend zu WO-A-97/03658) eine Vorrichtung zur Verabreichung von Vanillin umfassend Mull oder ein ähnliches Material, welches mit einer Vanillin freisetzenden Substanz getränkt ist, wobei der Mull in einem selbstklebenden Pflaster zum Aufbringen auf die Haut enthalten ist und wobei die Oberfläche des Pflasters, welche frei liegt, ein mittiges Loch aufweist, durch welches das Vanillin aus dem Mull austreten kann. Der Nachteil dieser Vorrichtung liegt jedoch darin, dass die Geschwindigkeit, mit der das Vanillin austritt, nicht ausreichend reguliert werden kann.

Ebenso beschreibt die WO-A-98/17262 eine Vorrichtung in Form eines Pflasters, jedoch zur transdermalen Verabreichung von Wirkstoffen, wobei hier der Wirkstoff wiederum auf ein Mull-ähnliches Material appliziert wird. Mittel zum transdermalen Verabreichen von Wirkstoffen unterliegen jedoch staatlicher Zulassungen. Darüber hinaus schränken Mull-ähnliche Materialien den Tragekomfort ein.

Im Übrigen sei noch auf die US-A-5455043, die DE-A-4433191, die DE-A-197 12 359 sowie die KR-A-200102288 hingewiesen.

Die der vorliegenden Erfindung zu Grunde liegende Aufgabe bestand darin, die sich aus dem Stand der Technik ergebenden Nachteile zu überwinden.

Zudem bestand die erfindungsgemäße Aufgabe darin, eine Vorrichtung bereitzustellen, mit deren Hilfe es möglich ist, Vanillin kontinuierlich im körpemahen Bereich freizusetzen, so dass dieses über die Nase aufgenommen werden kann.

Die vorstehenden Aufgaben wurden gelöst durch eine Vorrichtung zu kontinuierliche Freisetzung von Vanillin (3-Methoxy-4-hydroxybenzaldehyd) oder mindestens eines Vanillinderivats, vorzugsweise Ethylvanillin (3-Ethoxy-4-hydroxybenzaldehyd), oder deren Mischungen, umfassend eine für gasförmiges Vanillin oder für das gasförmige Vanillinderivat oder für deren gasförmige Mischungen impermeable Schicht, eine für gasförmiges Vanillin oder für das mindestens eine gasförmige Vanillinderivat oder für deren gasförmige Mischungen permeable Schicht sowie eine Zusammensetzung beinhaltend Vanillin oder das mindestens eine Vanillinderivat oder deren Mischungen, die zwischen diesen beiden Schichten lokalisiert ist. Die Vorrichtung wird im körpernahen Bereich eines Trägers, vorzugsweise durch Aufkleben auf die Haut des Trägers, eingesetzt. Bei dem Träger handelt es sich vorzugsweise um einen Menschen einen Hund, ein Schwein, einen Hamster, eine Katze, einen Hase, ein Kaninchen, wobei ein Mensch besonders bevorzugt ist.

Bei der für gasförmiges Vanillin, für gasförmiges Vanillinderivat oder für deren Mischungen impermeablen Schicht handelt es sich vorzugsweise um eine Folie beinhaltend Polyester, Polypropylen, Polyethylen, Ethylen-Propylen-Copolymere, Polyamide, wie Nylon-6,6, Metalle, beispielsweise Aluminium, Polyethylen, Teflon, Polycarbonat, Polyethylentherephthalat, Polybutyrat, Polyurethan oder Polyvinylchlorid, wobei eine Folie beinhaltend Polypropylen oder Polyethylen oder eine Folie beinhaltend ein Ethylen-Propylen-Copolymer besonders bevorzugt sind. Unter diesen Folien ganz besonders bevorzugt sind Polyethylenfolien, die unter der Handelbezeichung *Sclatrfilm*^{®} von DuPont, Canada, erhältlich sind. Die Dicke der für gasförmiges Vanillin, für gasförmige Vanillinderivative oder für deren Mischungen impermeablen Schicht wird vorzugsweise so gewählt, dass innerhalb eines Zeitintervalls von 24 Stunden höchstens 1 Gew.-%, bevorzugt höchstens 0,1 Gew.-% und darüber hinaus bevorzugt höchstens 0,01 Gew.-% der Zusammensetzung bei Raumtemperatur und einem Druck von 1 bar über diese Folie verdampfen können. Vorzugsweise weist diese Schicht eine Dicke in einem Bereich von 0,001 bis 1 mm, besonders bevorzugt in einem Bereich von 0,01 bis 0,5 mm und darüber hinaus bevorzugt in einem Bereich von 0,1 bis 0,25 mm auf.

Bei der für gasförmiges Vanillin, für gasförmiges Vanillinderivat oder für deren Mischungen permeablen Schicht handelt es sich vorzugsweise um eine Folie, welche die Eigenschaft aufweist, dass innerhalb eines Zeitintervalls von 24 Stunden beim Raumtemperatur und bei einem Druck von 1 bar mindestens 10 Gew.-%, bevorzugt mindestens 25 Gew.-% und darüber hinaus bevorzugt mindestens 50 Gew.-% des in der Zusammensetzung enthaltenen Vanillins, Ethylvanillins oder von deren Mischungen über diese Folie verdampfen können. Bevorzugte Folien sind Folien beinhaltend Polyethylen, Polyamid, Ethylen-Vinylacetat-Copolymer oder Mischungen dieser Monomere. Die permeable Schicht weist vorzugsweise eine Dicke in einem Bereich von 0,001 bis 1 mm, besonders bevorzugt in einem Bereich von 0,01 bis 0,5 mm und darüber hinaus bevorzugt in einem Bereich von 0,1 bis 0,25 mm auf. Es ist diesem Zusammenhang weiterhin bevorzugt, dass über die Wahl des Materials der permeablen Schicht und deren Dicke die in einem bestimmten Zeitintervalls bei einer definierten Temperatur und einem definierten Druck durch diese Schicht verdampfte Menge der Zusammensetzung reguliert werden kann. In einer besonderen Ausführungsform dieser Vorrichtung weist permeable Schicht mikroskopisch kleine Löcher mit einem Durchmesser in einem Bereich zwischen 1 und 100 Å, besonders bevorzugt in einem Bereich zwischen 10 und 50 Å aufweist, die vorzugsweise durch den Beschuss der Schicht mit Schweratomen, beispielsweise mit Bariumatomen, erhältlich ist. Durch die Lochdichte in der Folie, angegeben in der Anzahl der Löcher pro mm², und dem Durchmesser der Löcher kann ebenfalls die in einem bestimmten Zeitintervall bei einer definierten Temperatur und einem definierten Druck durch die permeable Schicht verdampfte Menge der Zusammensetzung reguliert werden.

Es ist in diesem Zusammenhang weiterhin bevorzugt, dass sowohl die permeable Schicht als auch die impermeabel Schicht eine Dicke von höchstens 0,1 mm, besonders bevorzugt von höchstens 0,01 mm aufweisen, so dass die erfindungsgemäße Vorrichtung nicht starr ist und sich bei Auflage auf eine unebene Fläche dieser Fläche anpassen kann.

Die Erfindung wird nun in ihren bevorzugten Ausführungsformen anhand einer nicht limitierenden Figur näher erläutert.

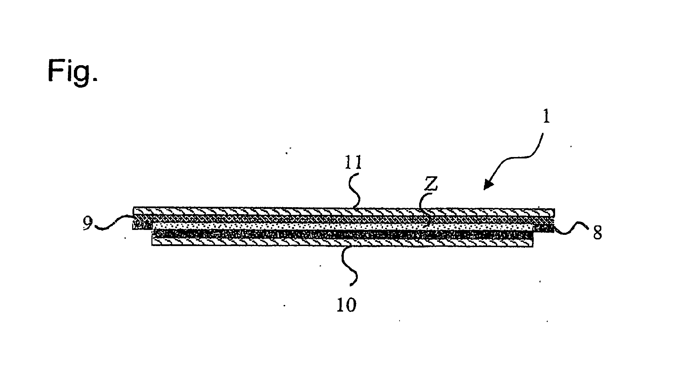

Die einzige Figur zeigt eine Ausführungsform der erfindungsgemäßen Vorrichtung in Form eines Pflasters als Querschnitt. Mit diesem Pflaster ist eine kontinuierliche Aufnahme von Vanillin, Ethylvanillin oder von deren Mischungen auf nasalem oder pulmonaler Weg möglich.

Wie dargestellt umfasst die als Pflaster ausgebildete Vorrichtung (1):
- eine für gasförmiges Vanillin oder für gasförmige Vanillinderivate oder für deren gasförmige Mischungen impermeablen Schicht (8),
- eine darüber liegende, für gasförmiges Vanillin oder für gasförmige Vanillinderivate oder für deren gasförmige Mischungen permeable Schicht (9) sowie die Zwischen beiden Schichten (8; 9) lokalisierte Zusammensetzung (Z),
   - wobei für gasförmiges Vanillin oder für gasförmige Vanillinderivate oder für deren gasförmige Mischungen impermeablen Schicht (8) an ihrer der permeablen Schicht (9) abgewandten Seite mit einem Klebstoff versehen ist,
   - eine erste, abdeckende Schutzschicht (10), die auf der mit dem Klebstoff versehenen Seite der impermeablen Schicht (8) haftet durch Abziehen mit der Hand, vorzugsweise durch Einwirkung einer Kraft von weniger als 10 N, besonders bevorzugt von weniger als 1 N und darüber hinaus bevorzugt von weniger als 0,1 N, von dieser Folie abgetrennt werden kann, wobei der Klebstoff auf impermeablen Schicht (8) verbleibt,
   - eine zweite, abdeckende Schutzschicht (11) aus einem für gasförmiges Vanillin oder für gasförmige Vanillinderivate oder für deren gasförmige Mischungen impermeablen Material, die auf der permeablen Schicht (9) an ihrer der impermeablen Schicht (8) abgewandten Seite klebend haftet und durch Abziehen mit der Hand, vorzugsweise durch Einwirkung einer Kraft von weniger als 10N, besonders bevorzugt von weniger als 1 N und darüber hinaus bevorzugt von weniger als 0,1 N, von der permeablen Schicht (9) abgetrennt werden kann.

Wird dieses Pflaster (1) in den köpernahen Bereich gebracht, beispielsweise durch das Aufkleben dieses Pflasters auf die Haut des Trägers nach dem Entfernen der ersten Schutzschicht (10), wobei die impermeable Schicht (8) in Kontakt mit der Haut tritt, so ermöglicht es die kontinuierliche Verdampfung der Zusammensetzung **(Z)** und somit die Aufnahme des Vanillins oder der Vanillinderivate oder von deren Mischungen von durch die Nase oder die Lunge des Trägers. Dies wird dadurch ermöglich, dass bedingt durch die Körpertemperatur des Trägers die Verdampfung der Zusammensetzung **(Z)** im Pflaster **(1)** und somit auch der Durchtritt des Vanillins oder der Vanillinderivate oder von deren Mischungen durch die permeable Schicht (9), nachdem die zweite, abdeckende Schutzschicht (11) entfernt worden ist, gefördert wird.

Dabei ist es bevorzugt, dass dieses Pflaster kein Mull oder ähnliche Materialien umfasst, die mit der Zusammensetzung getränkt sind. Es ist in diesem Zusammenhang weiterhin bevorzugt, das die Zusammensetzung eine dynamische Viskosität in einem Bereich von 10² bis 10⁶, besonders bevorzugt in einem Bereich von 10³ bis 10⁵ mPa·s bei 20°C nach DIN 53211 aufweist.

Bei dem in der erfindungsgemäßen Vorrichtung enthaltenen Klebstoff handelt es sich vorzugsweise um diejenigen, dem Fachmann bekannten Klebstoffe, die zum Anhaften eines Pflasters an die Haut eines Trägers eingesetzt werden. Bevorzugte Klebstoffe sind diejenigen, die von der Dow Corning Corporation, USA, unter der Handelbezeichnung BIO PSA angeboten werden, sowie Klebstoffe auf Acrylat-oder Siliconbasis, die für eine medizinische Verwendung zugelassen sind.

Bei den abdeckenden Schutzschichten handelt es sich vorzugsweise um Folien, welche die im Zusammenhang mit der impermeablen Schicht aufgeführten Materialen beinhalten. Auch die Dicke dieser Schutzschichten entspricht vorzugsweise derjenigen der permeablen oder impermeablen Schicht. Besonders bevorzugt werden als Schutzschichten Folien beinhaltend Polyethylen oder Polypropylen eingesetzt.

Die auf der permeablen Schicht klebend haftenden Schutzschichten dienen dazu, ein Verdampfen der Zusammensetzung aus der Vorrichtung zu verhindern, bevor diese zur Kontrollierten Freisetzung von Vanillin oder Vanillinderivaten oder von deren Mischungen eingesetzt wird. Diese Schutzschichten müssen demnach impermeabel für Vanillin oder Vanillinderivaten oder für deren Mischungen sein. Aus diesem Grund weisen sie vorzugsweise eine solche Dicke auf, dass innerhalb eines Zeitintervalls von 24 Stunden höchstens 1 Gew.-%, bevorzugt höchstens 0,1 Gew.-% und darüber hinaus bevorzugt höchstens 0,01 Gew.-% der Zusammensetzung bei Raumtemperatur und einem Druck von 1 bar über diese Schutzschicht verdampfen können.

Die in der erfindungsgemäßen Vorrichtung enthaltene Zusammensetzung basiert vorzugsweise auf
(α1) Vanillin oder Vanillinderivate oder deren Mischungen in einer Menge in einem Bereich von 0,1 bis 100 Gew.-%, vorzugsweise in einem Bereich von 1 bis 50 Gew.-% und darüber hinaus bevorzugt in einem Bereich von 5 bis 20 Gew.-%,
(α2) einen Träger in einer Menge in einem Bereich von 0 bis 99 Gew.-%, besonders bevorzugt in einem Bereich von 20 bis 70 Gew.-% und darüber hinaus
bevorzugt in einer Menge in einem Bereich von 40 bis 60 Gew.-%, sowie (α3) Hilfsstoffe in einer Menge in einem Bereich von 0 bis 50 Gew.-%, besonders bevorzugt in einer Menge in einem Bereich von 10 bis 40 Gew.-% und darüber hinaus bevorzugt in einer Menge in einem Bereich von 20 bis 30 Gew.-%,
wobei die Summe der Komponenten (α1) bis (α3) 100 Gew.-% beträgt.

Bei dem Träger handelt es sich vorzugsweise um einwertige Alkohle, beispielsweise Ethanol, mehrwertige Alkohole, beispielsweise Glycerin, Ethylenglykole oder deren Etherderivate, beispielsweise Diethylenglykol, Polyethylenglykol, Diethylenglykoldiethylether, Polyethylenglykoldimethylether, etherische Öle, beispielsweise Zedernholzöl, Zedernblätteröl, Thujaöl, Lavendelöl, Lavandinöl, Citronenöl, *Litsea cubeba-*Öl (May-Chang-Öl) oder Verty-Öl, Silikonöle, Wachse, Fette, beispielsweise Olivenöl, Erdnussöl oder Kokosfett, oder Mischungen aus mindestens zwei der vorstehend genannten Träger.

Als Hilfsstoffe sind vorzugsweise Substanzen enthalten, welche die Verdampfung des Vanillins, des Ethylvanillins oder die Verdampfung von deren Mischungen fördern. Bevorzugte, die Verdampfung fördernden Hilfsstoffe sind ausgewählt aus der Gruppe bestehend aus Hexylacetat, Isobutylacetat, Cyclohexylacetat, Prenylacetat, Isononylacetat, Isobornylacetat, Linalylacetat, Benzylacetat, Ethylbutyrat, Campher, Capronsäureethylester, Capronsäuremethylester, Carenen, Limonen, dem Terpenöl aus Orangen, Allylheptanoat, Methylhexenoat, Ethylisobutyrat, Methylpentylketon, Myrcen, Phellandren, Pinen, Butylvalerianat, Terpinolen, Paraffinölen oder Mischungen aus diesen mindestens zwei dieser Verbindungen. Als Hilfsstoffe sind weiterhin Farbstoffe, Konservierungsmitte, Viskositätsregler, UV-Stabilisatoren sowie weitere aromatische Verbindungen, beispielsweise Coumarin, bevorzugt. Weiterhin bevorzugt Hilfsstoffe sind von Vanillin oder Vanillinderivaten oder deren Mischungen verschiedene Duftstoffe, vorzugsweise ausgewählt aus der Gruppe bestehend aus Orange, Neroli, Jasmin, Melisse, Sandelholz, Bergamotte, Eichenmoos, Eukalyptus, Fenchel, Kamille, Kümmel, Salbei, Rosmarin, Sternanis, Teebaumöl, Weihrauch, Zimtrinde, Zirbelkiefer, Mandelöl, Jojoba oder Mischungen aus mindestens zwei davon. Wenn die Zusammensetzung in einer Vorrichtung zur transdermalen Verabreichung von Vanillin oder Vanillinderivaten oder von deren Mischungen eingesetzt wird, so enthält die Zusammensetzung als Hilfsstoffe vorzugsweise auch Penetrationsverstärker, beispielsweise Mischungen aus 1 -Menthol und Propylenglykol, eingesetzt werden.

Bevorzugt wird als Zusammensetzung eine Mischung aus Floral Vanillin (Ref. C6031/E Flora der A. Algto Ltd) und Ethanol eingesetzt. Zur Erhöhung der Viskosität kann diese Mischung zusätzlich Viskositätsregulatoren, beispielweise Glykol, enthalten.

Offenbart ist auch ein Verfahren zur Herstellung der vorstehend beschriebenen Vorrichtung zur kontrollierten Freisetzung von Vanillin, Ethylvanillin oder deren Mischungen.

Wenn die Vorrichtung in Form eines Pflasters ausgebildet ist, so erfolgt die Herstellung der erfindungsgemäßen Vorrichtung vorzugsweise dadurch, dass die Zusammensetzung zwischen die permeable und die impermeable Schicht eingebracht wird. Vorzugsweise geschieht dies dadurch, dass die Zusammensetzung zunächst auf ein definiertes Areal der permeable oder die impermeable Schicht aufgebracht wird und die andere Schicht über die Zusammensetzung gelegt wird, wobei die permeable und die impermeable Schicht an den Stellen, an denen sie unmittelbar miteinander in Kontakt treten, miteinander verbunden werden. Das Verbinden erfolgt dabei vorzugsweise durch Verschweißen oder Verkleben.

Die Offenbarung betrifft weiterhin die durch vorstehend beschriebenen Verfahren erhältlichen Vorrichtungen.

Gegenstand der vorliegenden Erfindung ist außerdem die Verwendung einer vorstehend beschriebenen Vorrichtung zur nasalen oder pulmonalen Aufnahme von Vanillin, Ethylvanillin oder von deren Mischungen zur Stimulation der Freisetzung von Serotonin im Zentralnervensystem.

Schließlich betrifft die vorliegende Erfindung auch die Verwendung einer vorstehend beschriebenen Vorrichtung in einem Diätplan oder in Kombination mit für eine bestimmte Diät ausgewähltem Nahrungsmittel. Beispielsweise wird die erfindungsgemäße Vorrichtung zusammen in einer Trennkostdiät in Kombination mit dafür geeigneten nur Eiweiß oder Kohlenhydrate enthaltenden Nahrungsmitteln eingesetzt. In einer anderen Diät wird die erfindungsgemäße Vorrichtung mit flüssiger Nahrung wie Frucht- und Gemüsesäften eingesetzt.

Die Erfindung wird nun anhand eines nicht limitierenden Beispiels näher erläutert.

### BEISPIEL

Bei einer Gruppe von 10 erwachsenen Testpersonen mit einem Boddy Mas Index zwischen 25 und 27 konnten bei einer Tragedauer von 45 Tagen pro Einheit eine Verringerung der Nahrungsaufnahme über einen Zeitraum von 31 Tagen beobachtet werden. Die Testpersonen nahmen im Mittel etwa 0,4 kg pro Tag ab.

## Patentansprüche

1. Eine Vorrichtung (1) in Form eines Pflasters zur kontinuierlichen Freisetzung von Vanillin und/oder Ethylvanillin, umfassend eine für gasförmiges Vanillin bzw. für gasförmiges Ethylvanillin impermeable Schicht (8), eine für gasförmiges Vanillin bzw. für gasförmiges Ethylvanillin permeable Schicht (9), sowie eine Zusammensetzung (Z) beinhaltend Vanillin bzw. Ethylvanillin, die zwischen diesen beiden Schichten (8; 9) lokalisiert ist, wobei die Vorrichtung (1) kein Mull umfasst, das mit der Zusammensetzung (Z) getränkt ist und wobei die impermeable Schicht (8) an ihrer der permeablen Schicht (9) abgewandten Seite mit einem Klebstoff zum Aufkleben der Vorrichtung (1) auf die Haut eines Trägers versehen ist.

2. Vorrichtung (1) nach Anspruch 1, wobei die Zusammensetzung (Z) eine dynamische Viskosität in einem Bereich von 10² bis 10⁶ mPas·s bei 20°C nach DIN 53211 aufweist.

3. Vorrichtung (1) nach Anspruch 1 oder 2, wobei die Zusammensetzung (Z) eine dynamische Viskosität in einem Bereich von 10³ bis 10⁵ mPas·s bei 20°C nach DIN 53211 aufweist.

4. Vorrichtung (1) nach einem der vorherigen Ansprüche, wobei die Zusammensetzung (Z) auf
(α1) Vanillin bzw. Ethylvanillin in einer Menge in einem Bereich von 0,1 bis 100 Gew.-%;
(α2) einem Träger in einer Menge in einem Bereich von 0 bis 99 Gew.-%; sowie
(α3) Hilfsstoffen in einer Menge in einem Bereich von 0 bis 50 Gew.-% basiert;
wobei die Summe der Komponenten (α1) bis (α3) 100 Gew.-% beträgt.

5. Vorrichtung (1) nach einem der vorherigen Ansprüche, wobei es sich bei der permeablen Schicht (9) um eine Folie handelt, die Löcher mit einem Durchmesser in einem Bereich zwischen 1 und 100 Å, insbesondere in einem Bereich zwischen 10 und 50 Å, aufweist.

6. Vorrichtung (1) nach einem der vorherigen Ansprüche, wobei es sich bei der permeablen Schicht (9) um eine Folie handelt, die Löcher mit einem Durchmesser und in einer solchen Lochdichte aufweist, dass innerhalb eines Zeitintervalls von 24 Stunden bei Raumtemperatur und bei einem Druck von 1 bar mindestens 10 Gew.-% des in der Zusammensetzung (Z) enthaltenen Vanillins bzw. Ethylvanillins verdampfen können.

7. Vorrichtung (1) nach Anspruch 6, wobei die Folie Löcher mit einem solchen Durchmesser und in einer solchen Lochdichte aufweist, dass innerhalb eines Zeitintervalls von 24 Stunden bei Raumtemperatur und bei einem Druck von 1 bar mindestens 25 Gew.-% des in der Zusammensetzung (Z) enthaltenen Vanillins bzw. Ethylvanillins verdampfen können.

8. Vorrichtung (1) nach Anspruch 7, wobei die Folie Löcher mit einem solchen Durchmesser und in einer solchen Lochdichte aufweist, dass innerhalb eines Zeitintervalls von 24 Stunden bei Raumtemperatur und bei einem Druck von 1 bar mindestens 50 Gew.-% des in der Zusammensetzung (Z) enthaltenen Vanillins bzw. Ethylvanillins verdampfen können.

9. Nicht medizinische Verwendung einer Vorrichtung (1), wie in einem der Ansprüche 1 bis 8 definiert, in einem Diätplan oder in Kombination mit für eine bestimmte Diät ausgewählte Nahrungsmittel.

10. Verwendung einer Vorrichtung (1), wie in einem der Ansprüche 1 bis 8 definiert, zur Herstellung eines Anzneimittels zur Stimulation der Freisetzung von Serotonin im Zentralnervensystem.

## Claims

1. A device (1) in a form of a patch for continuously releasing of vanillin compound and/or ethyl vanillin compound, comprising a layer impermeable (8) to said gaseous vanillin compound and/or gaseous ethyl vanillin compound, a layer permeable (9) to said gaseous vanillin compound and/or gaseous ethyl vanillin compound, and a composition (Z) comprising vanillin compound and/or ethyl vanillin compound, said composition (Z) being located between these two layers (8; 9), the device (1) not comprising lint impregnated with the composition (Z) and wherein the impermeable layer (8) on its side facing away from the permeable layer (9) is equipped with an adhesive for sticking the device (1) on the skin of a carrier.

2. The device (1) as claimed in claim 1, wherein the composition (Z) has a dynamic viscosity in a range from 10² to 10⁶ mPa s at 20 °C in accordance with DIN 53211.

3. The device (1) as claimed in claim 1 or 2, wherein the composition (Z) has a dynamic viscosity in a range from 10³ to 10⁵ mPa s at 20 °C in accordance with DIN 53211.

4. The device (1) as claimed in any one of the preceding claims, wherein the composition (Z) is based on
(α1) vanillin and/or ethyl vanillin in an amount in a range from 0,1 % to 100 % by weight;
(α2) a vehicle in an amount in a range from 0 % to 99 % by weight; and
(α3) excipients in an amount in a range from 0 % to 50 % by weight;
wherein the sum of components (α1) to (α3) being 100 % by weight.

5. The device (1) as claimed in any one of the preceding claims, wherein the permeable layer (9) comprises a film having holes having a diameter in a range between 1 and 100 Å, especially in a range between 10 and 50 Å.

6. The device (1) as claimed in any one of the preceding claims, wherein the permeable layer comprises a film having holes having a diameter, and a density, such that within a time interval of 24 hours at room temperature under a pressure of 1 bar at least 10 % by weight of the vanillin compound and/or ethyl vanillin compound present in the composition (Z) is able to evaporate.

7. The device (1) as claimed in claim 6, wherein the film having holes having a diameter, and a density, such that within a time interval of 24 hours at room temperature under a pressure of 1 bar at least 25 % by weight of the vanillin compound and/or ethyl vanillin compound present in the composition (Z) is able to evaporate.

8. The device (1) as claimed in claim 7, wherein the film having holes having a diameter, and a density, such that within a time interval of 24 hours at room temperature under a pressure of 1 bar at least 50 % by weight of the vanillin compound and/or ethyl vanillin compound present in the composition (Z) is able to evaporate.

9. The non-medical usage of a device (1), as defined in any one of the preceding claims 1 to 8, in a diet plan or in combination with a chosen food of a defined diet.

10. The usage of a device (1), as defined in any one of the preceding claims 1 to 8, for the production of medicine to stimulate the release of serotonin in the central nervous system.

## Revendications

1. Dispositif (1) sous forme d'un pansement adhésif pour le dégagement continue de vanilline et/ou vanilline éthylique, contenant une couche imperméable (8) pour vanilline gazeuse et/ou pour vanilline éthylique gazeuse, une couche perméable (9) pour vanilline gazeuse et/ou pour vanilline éthylique gazeuse, ainsi qu' une composition (Z) contenant vanilline et/ou vanilline éthylique, localisée entre ces deux couches (8; 9), cependant le dispositif (1) ne contient aucune gaze imbibée de la composition (Z) et la couche imperméable, à sa côtée détournée de la couche perméable (9), comporte un adhesif pour coller le dispositive (1) sur la peau d'un porteur.

2. Dispositif (1) selon la revendication 1, dans lequel la composition (Z) revêt une viscosité dynamique dans un cadre de 10² à 10⁶ mPas·s à 20°C conformément à DIN 53221.

3. Dispositif (1) selon la revendication 1 ou 2, dans lequel la composition (Z) revêt une viscosité dynamique dans un cadre de 10³ bis 10⁵ mPas·s à 20°C conformément à DIN 53221.

4. Dispositif (1) selon l'une des revendications précédées, dans lequel la composition (Z) se fonde sur
(α1) vanilline et/ou vanilline éthylique en quantité dans un cadre de 0,1 % à 100% de poids;
(α2) un porteur en quantité dans un cadre de von 0% à 99% de poids; ainsi que
(α3) substances auxiliaires en quantité dans un cadre de 0% à 50% de poids;
et en même temps la somme des composantes (α1) à (α3) s'élève à 100 % de poids.

5. Dispositif (1) selon l'une des revendications précédées, dans lequel la couche perméable (9) est un film qui revêt des trous d'un diamètre dans un cadre entre 1 et 100 Å, en particulier dans un cadre entre 10 et 50 Å.

6. Dispositif (1) selon l'une des revendications précédées, dans lequel la couche perméable (9) est un film qui revêt des trous d'un diamètre et de telle densité des trous, que dans un intervalle de temps de 24 heures à température ambiante et à une pression de 1 bar, au moins 10% de poids de la vanilline et/ou vanilline ethylique contenue dans la composition (Z) peuvent s'évaporer.

7. Dispositif (1) selon la revendication 6, dans lequel le film revêt des trous de tel diamètre et de telle densité des trous, que dans un intervalle de temps de 24 heures à température ambiante et à une pression de 1 bar, au moins 25% de poids de la vanilline et/ou vanilline ethylique contenue dans la composition (Z) peuvent s'évaporer.

8. Dispositif (1) selon la revendication 7, dans lequel le film revêt des trous de tel diamètre et de telle densité des trous, que dans un intervalle de temps de 24 heures à température ambiante et à une pression de 1 bar, au moins 50% de poids de la vanilline et/ou vanilline ethylique contenue dans la composition (Z) peuvent s'évaporer.

9. Utilisation non-médicale d'un dispositif (1), comme défini dans l'une des revendications 1 à 8, à un régime alimentaire ou combiné à des aliments choisis pour un certain régime.

10. Utilisation d'un dispositif (1), comme défini dans l'une des revendications 1 à 8, pour la production d'un médicament pour la stimulation du dégagement de sérotonine dans le système nerveux central.
